# EUROPEAN PATENT APPLICATION

(11) **EP 3 040 400 A1**
(43) Date of publication of application: **06.07.2016**
(21) Application number: 14200573.5
(22) Date of filing: 30.12.2014
(51) Int. Cl.: C10G 3/00, C07C 1/20

(54) **A METHOD OF STARTING UP AN OXYGENATE TO OLEFINS CONVERSION REACTION SYSTEM**

(71) Applicant: Shell Internationale Research Maatschappij B.V., 2596 HR Den Haag (NL)
(72) Inventor: CHEWTER, Leslie Andrew, 1031 HW Amsterdam (NL); MARSMAN, Jeroen Gerard, 1031 HW Amsterdam (NL); SANBORN, Richard Addison, Houston, TX 77082-3101 (US)
(74) Representative: Matthezing, Robert Maarten

(57) **Abstract**

A method of starting up an oxygenate to olefins conversion reaction system, where the system comprises a reaction section having one or more inlets and an outlet; one or more gas/solid separation devices, each having one inlet, a gas outlet and a catalyst outlet; and a quench section comprising a gas inlet, a quench medium inlet, a gas outlet and a quenched stream outlet wherein the reaction section outlet is in fluid communication with the gas/separation device inlet and the gas/separation device gas outlet is in fluid communication with the quench section gas inlet, said method comprising, in this order: a) passing a quench medium through the quench section by introducing the quench medium at the quench medium inlet and removing the quench medium at the quenched stream outlet; b) passing a gas stream through the reaction section by introducing the gas stream at one of the reaction section inlets and removing the gas at the reaction section outlet; c) passing the gas stream from the reaction section outlet to the gas/solid separation device inlet; and d) passing the gas stream through the gas/solid separation device and to the quench section.

## Description

### Field of the Invention

The invention relates to a reaction system for carrying out an oxygenate to olefins conversion reaction, and specifically to a method for starting up the reactor system.

### Background

Oxygenate-to-olefin processes are well described in the art. Typically, oxygenate-to-olefin processes are used to produce predominantly ethylene and propylene. An example of such an oxygenate-to-olefin process is described in US Patent Application Publication No. 2011/112344, which is herein incorporated by reference. The publication describes a process for the preparation of an olefin product comprising ethylene and/or propylene, comprising a step of converting an oxygenate feedstock in an oxygenate-to-olefins conversion system, comprising a reaction zone in which an oxygenate feedstock is contacted with an oxygenate conversion catalyst under oxygenate conversion conditions, to obtain a conversion effluent comprising ethylene and/or propylene.

Additional compounds, especially higher molecular weight hydrocarbons are typically produced with the ethylene and propylene in an oxygenate-to-olefins process. A method of improving the yield of lower molecular weight olefins is desired as these olefins, mainly ethylene and propylene, serve as feeds for the production of numerous chemicals. The catalyst and the activity of the catalyst is key to the performance of the reaction step.

### Summary of the Invention

The invention provides a method of starting up an oxygenate to olefins conversion reaction system, where the system comprises a reaction section having one or more inlets and an outlet; one or more gas/solid separation devices, each having one inlet, a gas outlet and a catalyst outlet; and a quench section comprising a gas inlet, a quench medium inlet, a gas outlet and a quenched stream outlet wherein the reaction section outlet is in fluid communication with the gas/separation device inlet and the gas/separation device gas outlet is in fluid communication with the quench section gas inlet, said method comprising, in this order: a) passing a quench medium through the quench section by introducing the quench medium at the quench medium inlet and removing the quench medium at the quenched stream outlet; b) passing a gas stream through the reaction section by introducing the gas stream at one of the reaction section inlets and removing the gas at the reaction section outlet; c) passing the gas stream from the reaction section outlet to the gas/solid separation device inlet; and d) passing the gas stream through the gas/solid separation device and to the quench section.

### Brief Description of the Drawings

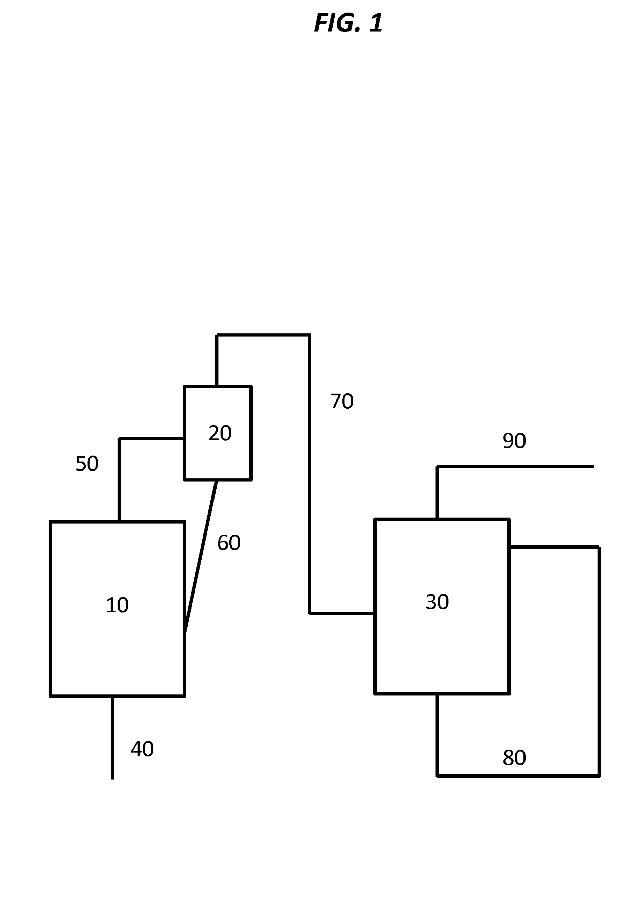

Figure 1 depicts an embodiment of a reaction system

### Detailed Description

This invention provides an improved startup process for an oxygenate to olefins conversion reaction system. The start-up method prevents entrained catalyst fines from being carried out of the quench section and/or plugging downstream equipment. In addition, if the gas stream is a heated gas stream then it may condensed in the quench tower, thus preventing the escape of hot gases to the atmosphere. This reduces the risk of safety incidents and ensures that fine particulate emissions do not exceed environmental regulations. If the heating medium is water (or steam), then it can be reheated and reused after being condensed in the quench section.

The oxygenate to olefins process receives as a feedstock a stream comprising one or more oxygenates. An oxygenate is an organic compound that contains at least one oxygen atom. The oxygenate is preferably one or more alcohols, preferably aliphatic alcohols where the aliphatic moiety has from 1 to 20 carbon atoms, preferably from 1 to 10 carbon atoms, more preferably from 1 to 5 carbon atoms and most preferably from 1 to 4 carbon atoms. The alcohols that can be used as a feed to this process include lower straight and branched chain aliphatic alcohols. In addition, ethers and other oxygen containing organic molecules can be used. Suitable examples of oxygenates include methanol, ethanol, n-propanol, isopropanol, methyl ethyl ether, dimethyl ether, diethyl ether, di-isopropyl ether, formaldehyde, dimethyl carbonate, dimethyl ketone, acetic acid and mixtures thereof. In a preferred embodiment, the feedstock comprises one or more of methanol, ethanol, dimethyl ether, diethyl ether or a combination thereof, more preferably methanol or dimethyl ether and most preferably methanol.

In one embodiment, the oxygenate is obtained as a reaction product of synthesis gas. Synthesis gas can, for example, be generated from fossil fuels, such as from natural gas or oil, or from the gasification of coal. In another embodiment, the oxygenate is obtained from biomaterials, such as through fermentation.

The oxygenate feedstock can be obtained from a pre-reactor, which converts methanol at least partially into dimethylether and water. Water may be removed, by e.g., distillation. In this way, less water is present in the process of converting oxygenates to olefins, which has advantages for the process design and lowers the severity of hydrothermal conditions to which the catalyst is exposed.

The oxygenate to olefins process, may in certain embodiments, also receive an olefin co-feed. This co-feed may comprise olefins having carbon numbers of from 1 to 8, preferably from 3 to 6 and more preferably 4 or 5. Examples of suitable olefin co-feeds include butene, pentene and hexene.

Preferably, the oxygenate feed comprises one or more oxygenates and olefins, more preferably oxygenates and olefins in an oxygenate:olefin molar ratio in the range of from 1000:1 to 1:1, preferably 100:1 to 1:1. More preferably, in a oxygenate:olefin molar ratio in the range of from 20:1 to 1:1, more preferably in the range of 18:1 to 1:1, still more preferably in the range of 15:1 to 1:1, even still more preferably in the range of 14:1 to 1:1. It is preferred to convert a C4 olefin, recycled from the oxygenate to olefins conversion reaction together with an oxygenate, to obtain a high yield of ethylene and propylene, therefore preferably at least one mole of oxygenate is provided for every mole of C4 olefin.

The olefin co-feed may also comprise paraffins. These paraffins may serve as diluents or in some cases they may participate in one or more of the reactions taking place in the presence of the catalyst. The paraffins may include alkanes having carbon numbers from 1 to 10, preferably from 3 to 6 and more preferably 4 or 5. The paraffins may be recycled from separation steps occurring downstream of the oxygenate to olefins conversion step.

The oxygenate to olefins process, may in certain embodiments, also receive a diluent co-feed to reduce the concentration of the oxygenates in the feed and suppress side reactions that lead primarily to high molecular weight products. The diluent should generally be non-reactive to the oxygenate feedstock or to the catalyst. Possible diluents include helium, argon, nitrogen, carbon monoxide, carbon dioxide, methane, water and mixtures thereof. The more preferred diluents are water and nitrogen with the most preferred being water.

The diluent may be used in either liquid or vapor form. The diluent may be added to the feedstock before or at the time of entering the reactor or added separately to the reactor or added with the catalyst. In one embodiment, the diluents is added in an amount in the range of from 1 to 90 mole percent, more preferably from 1 to 80 mole percent, more preferably from 5 to 50 mole percent, most preferably from 5 to 40 mole percent.

During the conversion of the oxygenates in the oxygenate to olefins conversion reactor, steam is produced as a by-product, which serves as an in-situ produced diluent. Typically, additional steam is added as diluent. The amount of additional diluent that needs to be added depends on the in-situ water make, which in turn depends on the composition of the oxygenate feed. Where the diluent provided to the reactor is water or steam, the molar ratio of oxygenate to diluent is between 10:1 and 1:20.

The oxygenate feed is contacted with the catalyst at a temperature in the range of from 200 to 1000 °C, preferably of from 300 to 800 °C, more preferably of from 350 to 700 °C, even more preferably of from 450 to 650°C. The feed may be contacted with the catalyst at a temperature in the range of from 530 to 620 °C, or preferably of from 580 to 610 °C. The feed may be contacted with the catalyst at a pressure in the range of from 0.1 kPa (1 mbar) to 5 MPa (50 bar), preferably of from 100 kPa (1 bar) to 1.5 MPa (15 bar), more preferably of from 100 kPa (1 bar) to 300 kPa (3 bar). Reference herein to pressures is to absolute pressures.

A wide range ofWHSV for the feedstock may be used. WHSV is defined as the mass of the feed (excluding diluents) per hour per mass of catalyst. The WHSV should preferably be in the range of from 1 hr⁻¹ to 5000 hr⁻¹.

The process takes place in a reactor and the catalyst may be present in the form of a fixed bed, a moving bed, a fluidized bed, a dense fluidized bed, a fast or turbulent fluidized bed, a circulating fluidized bed. In addition, riser reactors, hybrid reactors or other reactor types known to those skilled in the art may be used. In another embodiment, more than one of these reactor types may be used in series. In one embodiment, the reactor is a riser reactor. The advantage of a riser reactor is that it allows for very accurate control of the contact time of the feed with the catalyst, as riser reactors exhibit a flow of catalyst and reactants through the reactor that approaches plug flow.

The feedstocks described above are converted primarily into olefins. The olefins produced from the feedstock typically have from 2 to 30 carbon atoms, preferably from 2 to 8 carbon atoms, more preferably from 2 to 6 carbon atoms, most preferably ethylene and/or propylene. In addition to these olefins, diolefins having from 4 to 18 carbon atoms, conjugated or nonconjugated dienes, polyenes, vinyl monomers and cyclic olefins may be produced in the reaction.

In a preferred embodiment, the feedstock, preferably one or more oxygenates, is converted in the presence of a molecular sieve catalyst into olefins having from 2 to 6 carbon atoms. Preferably the oxygenate is methanol, and the olefins are ethylene and/or propylene.

The products from the reactor are typically separated and/or purified to prepare separate product streams in a recovery system. Such systems typically comprise one or more separation, fractionation or distillation towers, columns, and splitters and other associated equipment, for example, various condensers, heat exchangers, refrigeration systems or chill trains, compressors, knock-out drums or pots, pumps and the like.

The recovery system may include a demethanizer, a deethanizer, a depropanizer, a wash tower often referred to as a caustic wash tower and/or quench tower, absorbers, adsorbers, membranes, an ethylene-ethane splitter, a propylene-propane splitter, a butene-butane splitter and the like.

Typically in the recovery system, additional products, by-products and/or contaminants may be formed along with the preferred olefin products. The preferred products, ethylene and propylene are preferably separated and purified for use in derivative processes such as polymerization processes.

In addition to the propylene and ethylene, the products may comprise C4+ olefins, paraffins and aromatics that may be further reacted, recycled or otherwise further treated to increase the yield of the desired products and/or other valuable products. C4+ olefins may be recycled to the oxygenate to olefins conversion reaction or fed to a separate reactor for cracking. The paraffins may also be cracked in a separate reactor, and/or removed from the system to be used elsewhere or possibly as fuel.

Although less desired, the product will typically comprise some aromatic compounds such as benzene, toluene and xylenes. Although it is not the primary aim of the process, xylenes can be seen as a valuable product. Xylenes may be formed in the OTO process by the alkylation of benzene and, in particular, toluene with oxygenates such as methanol. Therefore, in a preferred embodiment, a separate fraction comprising aromatics, in particular benzene, toluene and xylenes is separated from the gaseous product and at least in part recycled to the oxygenate to olefins conversion reactor as part of the oxygenate feed. Preferably, part or all of the xylenes in the fraction comprising aromatics are withdrawn from the process as a product prior to recycling the fraction comprising aromatics to the oxygenate to olefins conversion reactor.

The C4+ olefins and paraffins formed in the oxygenate to olefins conversion reactor may be further reacted in an additional reactor containing the same or a different molecular sieve catalyst. In this additional reactor, the C4+ feed is converted over the molecular sieve catalyst at a temperature in the range of from 500 to 700 °C. The additional reactor is also referred to as an OCP reactor and the process that takes place in this reactor is referred to as an olefin cracking process. In contact with the molecular sieve catalyst, at least part of the olefins in the C4+ feed is converted to a product, which includes at least ethylene and/or propylene and preferably both. In addition to ethylene and/or propylene, the gaseous product may comprise higher olefins, i.e. C4+ olefins, and paraffins. The gaseous product is retrieved from the second reactor as part of a second reactor effluent stream.

The olefin feed is contacted with the catalyst at a temperature in the range of from 500 to 700 °C, preferably of from 550 to 650°C, more preferably of from 550 to 620°C, even more preferably of from 580 to 610°C; and a pressure in the range of from 0.1 kPa (1 mbara) to 5 MPa (50 bara), preferably of from 100 kPa (1 bara) to 1.5 MPa (15 bara), more preferably of from 100 kPa (1 bara) to 300 kPa (3 bara). Reference herein to pressures is to absolute pressures.

In one embodiment, the C4+ olefins are separated into at least two fractions: a C4 olefin fraction and a C5+ olefin fraction. In this embodiment, the C4 olefins are recycled to the oxygenate to olefins conversion reactor and the C5+ olefins are fed to the OCP reactor. The cracking behavior of C4 olefins and C5 olefins is believed to be different when contacted with a molecular sieve catalyst, in particular above 500 °C.

The cracking of C4 olefins is an indirect process which involves a primary oligomerisation process to a C8, C12 or higher olefin followed by cracking of the oligomers to lower molecular weight hydrocarbons including ethylene and propylene, but also, amongst other things, to C5 to C7 olefins, and by-products such as C2 to C6 paraffins, cyclic hydrocarbons and aromatics. In addition, the cracking of C4 olefins is prone to coke formation, which places a restriction on the obtainable conversion of the C4 olefins. Generally, paraffins, cyclics and aromatics are not formed by cracking. They are formed by hydrogen transfer reactions and cyclisation reactions. This is more likely in larger molecules. Hence the C4 olefin cracking process, which as mentioned above includes intermediate oligomerisation, is more prone to by-product formation than direct cracking of C5 olefins. The conversion of the C4 olefins is typically a function of the temperature and space time (often expressed as the weight hourly space velocity). With increasing temperature and decreasing weight hourly space velocity (WHSV) conversion of the C4 olefins in the feed to the OCP increases. Initially, the ethylene and propylene yields increase, but, at higher conversions, yield decreases at the cost of a higher by-product make and, in particular, a higher coke make, limiting significantly the maximum yield obtainable.

Contrary to C4 olefins, C5 olefin cracking is ideally a relatively straight forward-process whereby the C5 olefin cracks into a C2 and a C3 olefin, in particular above 500°C. This cracking reaction can be run at high conversions, up to 100%, while maintaining, at least compared to C4 olefins, high ethylene and propylene yields with a significantly lower by-product and coke make. Although, C5+ olefins can also oligomerise, this process competes with the more beneficial cracking to ethylene and propylene.

In a preferred embodiment of the process according to the present invention, instead of cracking the C4 olefins in the OCP reactor, the C4 olefins are recycled to the oxygenate toi olefins conversion reactor. Again without wishing to be bound by any particular theory, it is believed that in the oxygenate to olefins conversion reactor the C4 olefins are alkylated with, for instance, methanol to C5 and/or C6 olefins. These C5 and/or C6 olefins may subsequently be converted into at least ethylene and/or propylene. The main by-products from this oxygenate to olefins conversion reaction are again C4 and C5 olefins, which can be recycled to the oxygenate to olefins conversion reactor and olefin cracking reactor, respectively.

Therefore, preferably, where the gaseous products further include C4 olefins, at least part of the C4 olefins are provided to (i) the oxygenate to olefins conversion reactor together with or as part of the oxygenate feed, and/or (ii) the olefin cracking reactor as part of the olefin feed, more preferably at least part of the C4 olefins is provided to the oxygenate to olefins conversion reactor together with or as part of the oxygenate feed.

Preferably, where the gaseous products further include C5 olefins, at least part of the C5 olefins are provided to the olefin cracking reactor as part of the olefin feed. Preferably, the olefin feed to the olefin cracking reactor comprises C4+ olefins, preferably C5+ olefins, more preferably C5 olefins.

In a preferred embodiment, the oxygenate to olefins conversion reactor and the optional OCP reactor are operated as riser reactors where the catalyst and feedstock are fed at the base of the riser and an effluent stream with entrained catalyst exits the top of the riser. In this embodiment, gas/solid separators are necessary to separate the entrained catalyst from the reactor effluent. The gas/solid separator may be any separator suitable for separating gases from solids. Preferably, the gas/solid separator comprises one or more centrifugal separation units, preferably cyclone units, optionally combined with a stripper section.

The reactor effluent is preferably cooled in the gas/solid separator to terminate the conversion process and prevent the formation of by-products outside the reactors. The cooling may be achieved by use of a water quench.

Once the catalyst is separated from the effluent, the catalyst may be returned to the reaction zone from which it came, to another reaction zone or to a regeneration zone. Further, the catalyst that has been separated in the gas/solid separator may be combined with catalyst from other gas/solid separators before it is sent to a reaction zone or to the regeneration zone.

During conversion of the oxygenates to olefins, carbonaceous deposits known as "coke" are formed on the surface of and/or within the molecular sieve catalysts. To avoid a significant reduction in activity of the catalyst, the catalyst must be regenerated by burning off the coke deposits.

In one embodiment, a portion of the coked molecular sieve catalyst is withdrawn from the reactor and introduced into a regeneration system. The regeneration system comprises a regenerator where the coked catalyst is contacted with a regeneration medium, preferably an oxygen-containing gas, under regeneration temperature, pressure and residence time conditions.

Examples of suitable regeneration media include oxygen, O₃, SO₃, N₂O, NO, NO₂, N₂O₅, air, air enriched with oxygen, air diluted with nitrogen or carbon dioxide, oxygen and water, carbon monoxide and/or hydrogen. The regeneration conditions are those capable of burning at least a portion of the coke from the coked catalyst, preferably to a coke level of less than 75% of the coke level on the catalyst entering the regenerator. More preferably the coke level is reduced to less than 50% of the coke level on the catalyst entering the regenerator and most preferably the coke level is reduced to less than 30% of the coke level on the catalyst entering the regenerator. Complete removal of the coke is not necessary as this may result in degradation of the catalyst.

The regeneration temperature is in the range of from 200 °C to 1500 °C, preferably from 300 °C to 1000 °C, more preferably from 450 °C to 700 °C and most preferably from 500 °C to 700 °C. In a preferred embodiment, the catalyst is regenerated at a temperature in the range of from 550 to 650 °C.

The preferred residence time of the coked molecular sieve catalyst in the regenerator is in the range of from 1 minute to several hours, most preferably 1 minute to 100 minutes. The preferred volume of oxygen in the regeneration medium is from 0.01 mole percent to 10 mole percent based on the total volume of the regeneration medium.

In one embodiment, regeneration promoters, typically metal containing compounds such as platinum and palladium are added to the regenerator directly or indirectly, for example with the coked catalyst composition. In another embodiment, a fresh molecular sieve catalyst is added to the regenerator.

In an embodiment, a portion of the regenerated molecular sieve catalyst from the regenerator is returned to the reactor, directly to the reaction zone or indirectly by pre-contacting with the feedstock.

The burning of coke is an exothermic reaction and in certain embodiments, the temperature in the regeneration system is controlled to prevent it from rising too high. Various known techniques for cooling the system and/or the regenerated catalyst may be employed including feeding a cooled gas to the regenerator, or passing the regenerated catalyst through a catalyst cooler. A portion of the cooled regenerated catalyst may be returned to the regenerator while another portion is returned to the reactor.

In certain embodiments, there is not sufficient coke on the catalyst to raise the temperature of the catalyst to desired levels. In one embodiment, a liquid or gaseous fuel may be fed to the regenerator where it will combust and provide additional heat to the catalyst.

Catalysts suitable for use in the conversion of oxygenates to olefins may be made from practically any small or medium pore molecular sieve. One example of a suitable type of molecular sieve is a zeolite. Suitable zeolites include, but are not limited to AEI, AEL, AFT, AFO, APC, ATN, ATT, ATV, AWW, BIK, CAS, CHA, CHI, DAC, DDR, EDI, ERI, EUO, FER, GOO, HEU, KFI, LEV, LOV, LTA, MFI, MEL, MON, MTT, MTW, PAU, PHI, RHO, ROG, THO, TON and substituted forms of these types. Suitable catalysts include those containing a zeolite of the ZSM group, in particular of the MFI type, such as ZSM-5, the MTT type, such as ZSM-23, the TON type, such as ZSM-22, the MEL type, such as ZSM-11, and the FER type. Other suitable zeolites are for example zeolites of the STF-type, such as SSZ-35, the SFF type, such as SSZ-44 and the EU-2 type, such as ZSM-48. Preferred zeolites for this process include ZSM-5, ZSM-22 and ZSM-23.

A suitable molecular sieve catalyst may have a silica-to-alumina ratio (SAR) of less than 280, preferably less than 200 and more preferably less than 100. The SAR may be in the range of from 10 to 280, preferably from 15 to 200 and more preferably from 20 to 100.

A preferred MFI-type zeolite for the oxygenate to olefins conversion catalyst has a silica-to-alumina ratio, SAR, of at least 60, preferably at least 80. More preferred MFI-type zeolite has a silica-to-alumina ratio, SAR, in the range of 60 to 150, preferably in the range of 80 to 100.

The zeolite-comprising catalyst may comprise more than one zeolite. In that case it is preferred that the catalyst comprises at least a more-dimensional zeolite, in particular of the MFI type, more in particular ZSM-5, or of the MEL type, such as zeolite ZSM-11, and a one-dimensional zeolite having 10-membered ring channels, such as of the MTT and/or TON type.

It is preferred that zeolites in the hydrogen form are used in the zeolite-comprising catalyst, e.g., HZSM-5, HZSM-11, and HZSM-22, HZSM-23. Preferably at least 50wt%, more preferably at least 90wt%, still more preferably at least 95wt% and most preferably 100wt% of the total amount of zeolite used is in the hydrogen form. It is well known in the art how to produce such zeolites in the hydrogen form.

Another example of suitable molecular sieves are siliocoaluminophosphates (SAPOs). SAPOs have a three dimensional microporous crystal framework of PO2+, AlO2-, and SiO2 tetrahedral units. Suitable SAPOs include SAPO-17, -18, 34, -35, -44, but also SAPO-5, -8, -11, -20, -31, -36, 37, -40, -41, -42, -47 and -56; aluminophosphates (AlPO) and metal substituted (silico)aluminophosphates (MeAlPO), wherein the Me in MeAlPO refers to a substituted metal atom, including metal selected from one of Group IA, IIA, IB, IIIB, IVB, VB, VIB, VIIB, VIIIB and lanthanides of the Periodic Table of Elements. Preferred SAPOs for this process include SAPO-34, SAPO-17 and SAPO-18. Preferred substituent metals for the MeAlPO include Co, Cr, Cu, Fe, Ga, Ge, Mg, Mn, Ni, Sn, Ti, Zn and Zr.

The molecular sieves described above are formulated into molecular sieve catalyst compositions for use in the oxygenates to olefins conversion reaction and the olefin cracking step. The molecular sieves are formulated into catalysts by combining the molecular sieve with a binder and/or matrix material and/or filler and forming the composition into particles by techniques such as spray-drying, pelletizing, or extrusion. The molecular sieve may be further processed before being combined with the binder and/or matrix. For example, the molecular sieve may be milled and/or calcined.

Suitable binders for use in these molecular sieve catalyst compositions include various types of aluminas, aluminophosphates, silicas and/or other inorganic oxide sol. The binder acts like glue binding the molecular sieves and other materials together, particularly after thermal treatment. Various compounds may be added to stabilize the binder to allow processing.

Matrix materials are usually effective at among other benefits, increasing the density of the catalyst composition and increasing catalyst strength (crush strength and/or attrition resistance). Suitable matrix materials include one or more of the following: rare earth metals, metal oxides including titania, zirconia, magnesia, thoria, beryllia, quartz, silica or sols, and mixtures thereof, for example, silica-magnesia, silica-zirconia, silica-titania, and silica-alumina. In one embodiment, matrix materials are natural clays, for example, kaolin. A preferred matrix material is kaolin.

In one embodiment, the molecular sieve, binder and matrix material are combined in the presence of a liquid to form a molecular sieve catalyst slurry. The amount of binder is in the range of from 2 to 40 wt%, preferably in the range of from 10 to 35 wt%, more preferably in the range of from 15 to 30 wt%, based on the total weight of the molecular sieve, binder and matrix material, excluding liquid (after calcination).

After forming the slurry, the slurry may be mixed, preferably with rigorous mixing to form a substantially homogeneous mixture. Suitable liquids include one or more of water, alcohols, ketones, aldehydes and/or esters. Water is the preferred liquid. In one embodiment, the mixture is colloid-milled for a period of time sufficient to produce the desired texture, particle size or particle size distribution.

The molecular sieve, matrix and optional binder can be in the same or different liquids and are combined in any order together, simultaneously, sequentially or a combination thereof. In a preferred embodiment, water is the only liquid used.

In a preferred embodiment, the slurry is mixed or milled to achieve a uniform slurry of sub-particles that is then fed to a forming unit. A slurry of the zeolite may be prepared and then milled before combining with the binder and/or matrix. In a preferred embodiment, the forming unit is a spray dryer. The forming unit is typically operated at a temperature high enough to remove most of the liquid from the slurry and from the resulting molecular sieve catalyst composition. In a preferred embodiment, the particles are then exposed to ion-exchange using an ammonium nitrate or other appropriate solution.

In one embodiment, the ion exchange is carried out before the phosphorous impregnation. The ammonium nitrate is used to ion exchange the zeolite to remove alkali ions. The zeolite can be impregnated with phosphorous using phosphoric acid followed by a thermal treatment to H+ form. In another embodiment, the ion exchange is carried out after the phosphorous impregnation. In this embodiment, alkali phosphates or phosphoric acid may be used to impregnate the zeolite with phosphorous, and then the ammonium nitrate and heat treatment are used to ion exchange and convert the zeolite to the H+ form.

Alternatively to spray drying the catalyst may be formed into spheres, tablets, rings, extrudates or any other shape known to one of ordinary skill in the art. The catalyst may be extruded into various shapes, including cylinders and trilobes.

The average particle size is in the range of from 1-200 µm, preferably from 50-100 µm. If extrudates are formed, then the average size is in the range of from 1 mm to 10 mm, preferably from 2 mm to 7 mm.

The catalyst may further comprise phosphorus as such or in a compound, i.e. phosphorus other than any phosphorus included in the framework of the molecular sieve. It is preferred that a MEL or MFI-type zeolite comprising catalyst additionally comprises phosphorus.

The molecular sieve catalyst is prepared by first forming a molecular sieve catalyst precursor as described above, optionally impregnating the catalyst with a phosphorous containing compound and then calcining the catalyst precursor to form the catalyst. The phosphorous impregnation may be carried out by any method known to one of skill in the art.

The phosphorus-containing compound preferably comprises a phosphorus species such as PO₄³⁻, P-(OCH₃)₃, or P₂O₅, especially PO₄³⁻. Preferably the phosphorus-containing compound comprises a compound selected from the group consisting of ammonium phosphate, ammonium dihydrogen phosphate, dimethylphosphate, metaphosphoric acid and trimethyl phosphite and phosphoric acid, especially phosphoric acid. The phosphorus containing compound is preferably not a Group II metal phosphate. Group II metal species include magnesium, calcium, strontium and barium; especially calcium.

In one embodiment, phosphorus can be deposited on the catalyst by impregnation using acidic solutions containing phosphoric acid (H₃PO₄). The concentration of the solution can be adjusted to impregnate the desired amount of phosphorus on the precursor. The catalyst precursor may then be dried.

The catalyst precursor, containing phosphorous (either in the framework or impregnated) is calcined to form the catalyst. The calcination of the catalyst is important to determining the performance of the catalyst in the oxygenate to olefins process.

The calcination may be carried out in any type of calciner known to one of ordinary skill in the art. The calcination may be carried out in a tray calciner, a rotary calciner, or a batch oven optionally in the presence of an inert gas and/or oxygen and/or steam

The calcination may be carried out at a temperature in the range of from 400 °C to 1000 °C, preferably in a range of from 450 °C to 800 °C, more preferably in a range of from 500 °C to 700 °C. Calcination time is typically dependent on the degree of hardening of the molecular sieve catalyst composition and the temperature and ranges from about 15 minutes to about 2 hours.

The calcination temperatures described above are temperatures that are reached for at least a portion of the calcination time. For example, in a rotary calciner, there may be separate temperature zones that the catalyst passes through. For example, the first zone may be at a temperature in the range of from 100 to 300 °C. At least one of the zones is at the temperatures specified above. In a stationary calciner, the temperature is increased from ambient to the calcination temperatures above and so the temperature is not at the calcination temperature for the entire time.

In a preferred embodiment, the calcination is carried out in air at a temperature of from 500 °C to 600 °C. The calcination is carried out for a period of time from 30 minutes to 15 hours, preferably from 1 hour to 10 hours, more preferably from 1 hour to 5 hours.

The calcination is carried out on a bed of catalyst. For example, if the calcination is carried out in a tray calciner, then the catalyst precursor added to the tray forms a bed which is typically kept stationary during the calcination. If the calcination is carried out in a rotary calciner, then the catalyst added to the rotary drum forms a bed that although not stationary does maintain some form and shape as it passes through the calciner.

The startup method will be described with regards to an embodiment of a oxygenate conversion reaction system depicted in Figure 1. The reaction system comprises a reaction zone 10, a gas/solids separation device 20 and a quench section 30. The method comprises first, passing a quench medium through the quench section 30 by introducing the quench medium at the quench medium inlet(s) and removing the quench medium at the quenched stream outlet. The quench medium flows can be circulated via line 80 from the quenched stream outlet of the quench section 30 and back into the inlet of the quench section. Heat exchangers and/or pumps may be located along line 80 to cool and/or pump the quench medium in the circulation loop.

The method comprises, next, passing a gas stream through the reaction section 10 by introducing the gas stream via line 40 through a reaction section inlet and removing the gas stream at the reaction section outlet and into line 50. The gas stream is passed via line 50 into the gas/solid separation device. The gas/solid separation device separates the gas from entrained catalyst and returns the catalyst via line 60 to the reactor. The gas is passed via line 70 to the quench section where it is contacted with the quench medium.

The reaction system can contain additional sections and variations of the lineup described above with respect to Figure 1, some of which will be further described herein. The quench section can be any vessel or conduit that provides a contacting zone for contacting of a gas with a quench medium. The quench section may comprise a spray tower where the quench medium is sprayed from one or more injection points in the spray tower. The quench section may be a quench tower comprising one or more sets of internals that facilitate contacting between the gas stream and the quench medium. The quench section may be a direct quench fitting that is a conduit comprising injection points along the length of the conduit for injecting the quench medium. The quench section may also consist of a combination of these options.

The quench medium may be an aqueous or hydrocarbon stream. The quench medium may be any stream that cools the gas stream.

The reaction section may comprise any type of reactor, but the invention is especially useful for reactors that contain a fluidized catalyst, and specifically a finely divided, fluidizable catalyst. The reactor may comprise, for example, a fluidized bed, a turbulent fluidized bed, a fast or circulating fluidized bed or a riser reactor. The reaction section may comprise a combination of the above reactor types.

The reaction section may contain catalyst before the gas stream is passed through the reaction section or it may be added to the reactor after the gas stream has begun to flow through the reaction section. In one embodiment, the startup method is used to startup a system that has been used before and already contains catalyst.

The gas stream may be passed through the reaction system at a velocity high enough to fluidize the catalyst located in the reaction section. The gas stream may comprise air, nitrogen, steam or a combination thereof. The gas stream may also comprise a process feed stream that may contain oxygenates, olefins or other hydrocarbons. The gas stream may be heated before entering the reaction section so that it raises the temperature of the reaction section and/or the catalyst present in the reaction section.

The gas stream may entrain a portion of the catalyst as it passes out of the reaction section. A portion of this entrained catalyst may be separated in the gas/solid separation device. The catalyst may be returned to the reactor or to a regeneration section which will be described in more detail below. The gas/solid separation device is preferably a cyclone.

The gas stream is then passed to the quench section. The quench section can be used to condense any condensable gases in the gas stream and/or to remove any solids that are entrained in the gas stream. If the gas stream is condensed in the quench section, it may be heated outside of the quench section and recycled to the reactor for further heating. For example, if steam is used as the gas stream, then it can be condensed in the quench section. Outside of the quench section it can be heated and/or vaporized again and then recycled to the reactor to provide additional heat.

The method may include passing an additional heating medium to the reaction section after it has been heated to a desired temperature. For example, the reaction section may be heated with hot air to a first temperature, then heated with steam to a second temperature. In another example, steam at different temperatures may be used to heat the reactor to a final desired temperature. In another embodiment, hot air or steam may be used to heat the reaction section to a first temperature, and then heated catalyst may be added to the reaction section to further raise the temperature of the reaction section.

The reaction system may also comprise a regeneration section. During operation of the reaction system, the regeneration section is used to regenerate the catalyst by removing coke. During startup, catalyst may be added to the regeneration section. The catalyst may be heated in the regeneration section and then the heated catalyst may be passed to the reaction section as a heating medium. The catalyst in the regeneration section may be heated by contacting it with a heated gas stream. In another embodiment, the catalyst may be heated by adding fuel to the regeneration and combusting the fuel inside the regeneration section. The catalyst may be circulated between the reaction section and the regeneration section to continue to heat the reaction section by heating the catalyst in the regeneration section.

A heated gas stream used to heat the catalyst in the regeneration section and/or combustion products leaving the regeneration section may be passed to a gas/solid separation device, heat recovery equipment and then to the atmosphere.

## Claims

1. A method of starting up an oxygenate to olefins conversion reaction system, where the system comprises a reaction section having one or more inlets and an outlet; one or more gas/solid separation devices, each having one inlet, a gas outlet and a catalyst outlet; and a quench section comprising a gas inlet, a quench medium inlet, a gas outlet and a quenched stream outlet wherein the reaction section outlet is in fluid communication with the gas/solid separation device inlet and the gas/separation device gas outlet is in fluid communication with the quench section gas inlet, said method comprising, in this order:
a. passing a quench medium through the quench section by introducing the quench medium at the quench medium inlet and removing the quench medium at the quenched stream outlet;
b. passing a gas stream through the reaction section by introducing the gas stream at one of the reaction section inlets and removing the gas at the reaction section outlet;
c. passing the gas stream from the reaction section outlet to the gas/solid separation device inlet; and
d. passing the gas stream through the gas/solid separation device and to the quench section.

2. The method of claim 1 wherein the quench section is any vessel or conduit that provides a contacting zone for contacting of a gas with a quench medium.

3. The method of claim 2 wherein the quench section comprises a spray tower, a quench tower comprising internals or a direct quench fitting.

4. The method of any of claims 1-3 wherein the reaction section comprises a fluidized bed, turbulent fluidized bed, fast or circulating fluidized bed, riser reactor or a combination thereof

5. The method of any of claims 1-4 wherein the reaction system contains catalyst.

6. The method of claim 5 wherein the gas stream is passed at a sufficient velocity to fluidize the catalyst particles in the reaction system.

7. The method of any of claims 1-6 further comprising adding catalyst to the reaction section.

8. The method of claim 7 wherein the catalyst is added after step d).

9. The method of any of claims 1-8 wherein the gas stream is a heated gas stream that is used to increase the temperature of one or more sections of the reaction section system.

10. The method of any of claims 1-9 wherein the gas stream comprises air, nitrogen or steam.

11. The method of any of claims 1-10 wherein the gas stream comprises a process feed stream.

12. The method of any of claims 1-11 wherein the quench medium is an aqueous or hydrocarbon stream.

13. The method of any of claims 1-12 wherein the gas stream entrains an amount of catalyst as it exits the reaction section.

14. The method of claim 13 wherein at least a portion of the entrained catalyst is separated from the gas stream in the gas/solid separation device.

15. The method of any of claims 13-14 wherein at least a portion of the entrained catalyst is passed with the gas stream to the quench section.

16. The method of any of claims 1-15 wherein the gas/solid separation device is a cyclone.

17. The method of any of claims 1-16 further comprising passing an additional heating medium into the reaction section once the reaction section is heated to a first desired temperature until the reaction section is heated to a second desired temperature.

18. The method of any of claims 1-17 wherein the gas stream is condensed in the quench section.

19. The method of claim 18 further comprising heating the condensed gas stream and recycling it to the reaction section to provide heat.

20. The method of any of claims 1-19 wherein the reaction system also comprises a regeneration section.

21. The method of claim 20 further comprising:
a. adding catalyst to the regeneration section;
b. heating the catalyst in the regeneration section; and
c. passing the heated catalyst to the reaction section.

22. The method of claim 21 wherein a gas stream is fed to the regeneration section at a velocity sufficient to fluidize the catalyst particles in the regeneration section

23. The method of claim 21 wherein the catalyst in the regeneration section is heated by combustion of a fuel.

24. The method of claim 21 wherein the catalyst in the regeneration section is heated by passing a regenerator heating medium into the regeneration section.

25. The method of claim 24 further comprising passing the regenerator heating medium from the regeneration section to a gas/solid separation device.

26. The method of any of claims 21-25 further comprising circulating the catalyst between the reaction section and the regeneration section.

27. The method of any of claims 1-26 wherein the reaction system also comprises a product recovery section in fluid communication with the quench section.

28. The method of any of claims 1-27 wherein the reaction section system is being restarted following a shutdown and the catalyst was added to the reaction section before the prior startup.
